# EUROPEAN PATENT APPLICATION

(11) **EP 4 001 397 A1**
(43) Date of publication of application: **25.05.2022**
(21) Application number: 20461583.5
(22) Date of filing: 19.11.2020
(51) Int. Cl.: C12M 3/00, C12M 1/12, C12M 1/36

(54) **CUVETTE, CASSETTE AND SYSTEM FOR MACROSCALE 3D GROWTH AND TREATMENT OF CELLS**

(71) Applicant: Real Research Sp. z o.o., 30-348 Kraków (PL)
(72) Inventor: Krzykawski, Marcin, 34-120 Andrychow (PL); Krzykawska, Renata, 34-120 Andrychow (PL); Earnshaw, David, 30-364 Krakow (PL)
(74) Representative: Wazynska, Miroslawa

(57) **Abstract**

The subject of the invention is a cuvette (3) for 3D cell culture growth comprising a main container (5) comprising means for macroscale 3D growth of cells and two end covers (4) at inlet and outlet ends of the main container (5), connected with cell culture media input and output tubing, wherein fluid transportation ports (7) placed at the inlet and outlet end covers (4). Another aspect of the invention is a cassette (2) for growth and treatment of cells comprising at least one container for cell culture growth, a cell culture media reservoir, a reagent reservoir, a waste container, and interconnecting tubes enabling connection with peristaltic pumps, wherein the cassette (2) is detachable and movable as an entity. Third aspect of the invention is an automated reagent delivery system for growth and treatment of cells, wherein the system comprises at least one slide out drawer (1), at least two peristaltic pumps attached to the drawer, the cassette (2) according to the invention attached to the drawer (1) and connected with the peristaltic pumps, a computer controller and computer program product (15) comprising means for manual and automatic control of liquid flow rates, output composition between the independent liquid reservoirs, pump control and execution of preset liquid flow programs, customisation and saving of new automated liquid handling programs.

## Description

The subject of the invention is a cuvette for macroscale 3D growth and treatment of cells, a cassette for growth and treatment of cells and a system for continuous, long-term cell growth and treatment, controlled by a computer application. The system is primarily used to confirm whether a given drug actually works in in vitro therapy, and to perform drug tests on cell culture and to prepare a drug concentration curve. The goal of the drug concentration curve is to mimic the in vivo ADME process of drug: absorption, digestion, metabolism, excretion. The device is to be used especially in the case of testing drugs used in the treatment of cancer cells or other disease models. Other large scale cell culture applications can be also explored like, for example, artificial meat cultivation.

Cancer is still the leading cause of death in the world. High morphological and phenotypic heterogeneity of the population of cancer cells, as well as their ability to develop genetic and epigenetic mutations increasing the prevalence of chemo-resistance are the main causes of failure of anticancer therapies. The occurrence of variability between the same types of cancer among patients makes it difficult to choose not only the right treatment regimens, but also to determine the effective dose of drugs. In addition, the available molecular biology methods that allow personalization of the treatment path for individual patients are not as effective as desired due to the time interval in generating data, which often leads to the development of secondary resistance to drugs and the formation of metastases. Given the above, the search for alternative methods that facilitate the selection of individual therapies is a sensible approach in the development of anticancer medicines. However, there remains one critical challenge that faces the oncology research and clinician communities in choosing the best chemotherapy regimen in addition to the complexities of cancer heterogeneity described above. This challenge concerns the disappointment when drugs shown to be effective against tumour cells in laboratory in vitro tests are often much less effective in the clinic. While part of this failure in drug efficacy is likely due to the differences in drug concentrations that tumour cells get exposed to in in vitro compared with in vivo scenarios, the larger cause of efficacy failure is very likely due to the substantial differences in cell and tissue organisation and diversity, gene expression and metabolic state found in patient tumours compared with the highly simplified 2D monolayer culturing commonly used in the drug discovery and characterisation process.

Examples of prior art solutions are known from documents such as EP3115449, WO2012033439, US20110217771 or EP1458483.

There is a growing need for 3D cell culture instead of 2D culture in order to begin to recreate the cell morphologies, behaviours, gene expression and metabolic profiles found in vivo.

There are different types of prior art receptacles or surfaces that have been developed for 3D cell culture including, for example: conventional petri dishes, conventional multi-well plates (6-well to 384-well), solid carrier beads or mats used inside walled containers, or modified glass or plastic microscope slides.

Also "Large Scale Imaging by Fine Spatial Alignment of Multi-Scanning Data with Gel Cube Device" M. Hagiwara et al. Appl. Sci. 2018, 8(2), 235 discloses 3D culture platform enabling large scale 3D imaging by fine spatial alignment of the image dataset obtained from multiple directions.

There are prior art designs that enable the supply of cell culture media and additional reagents to growing 3D cell cultures (and not just batch-wise replenishment of surface covering cell culture media), such as: synthetic hollow fibres (can be in layers or matrix layout) for supplying nutrients to microscope slide sized cultures; rolled wall vessels (creating micro zero gravity environment) containing 3D solid scaffold mat pieces supplied with pumped cell culture media; synthetic hollow fibres wrapped up 'swiss-roll' style with solid 3D scaffold mats inside a cylindrical vessel supplied with pumped cell culture media; sealed vessels containing microscope slide sized cultures through which cell culture media can be pumped and which provide for additional cell types to be grown in adjoining diffusion linked chambers; conventional continuous-flow stirred bioreactors that in principle can be used with cells grown on 3D scaffold coated beads, matting or grown encapsulated within protective gel beads, cell culture media pumped through extracellular matrix composed tubes contained within a vessel; supply and analysis of used cell culture media supplied by micro-diffusion channels constructed within microscope slide sized apparatus.

Examples of prior art supports and containers for cell cultures growth are known from: WO2015044813, EP3115449, WO2012033439, US20110217771, EP3019625, US20140141464, US20160139110 or EP1458483.

Prior art documents CN103077296 A and CN103077295 A concern a problem of simulating intravenous injection and oral administration pharmacokinetics model based on flow velocity regulation. These systems are based on pumping the fluids with one peristaltic pump and then mixing all components according to complicated dilution mathematical schemes. As a result the presented approach is very complicated.

None of the known above technologies provide way of using 3D-matrices in hardware devices capable of supporting and monitoring 3D cell growth in a long term, researcher friendly manner. There is also no prior art giving at the same time a possibility to mimic real-life pharmacokinetics of a tested drug type. Most of prior art documents focus on microscale experiments, and as it well known microscale does not always reliably reflects the phenomena occurring in macroscale.

The present invention addresses several aspects of the challenges listed above.

Firstly, in enabling the programming of clinically meaningful drug concentration dosing profiles and timings to cell cultures in a researcher friendly manner.

Secondly, in creating 3D cell culture environments at a larger scale than normal multiwell plates or microscope-like type slides for example EP3020480A1, and thereby encouraging the growth of heterogeneous cell populations with morphologies, organisation, gene expression and metabolic activities much closer to those in clinically relevant tumours. The goal of the invention is to provide a 3D cell culture system which will allow high level experiments like for example simulation of drug concentrations for experiments on dosing or drug metabolism and pharmacokinetics over real life long periods of time. For the microscope slide sized culture surfaces described above, there is the obvious limitation of culture scaffold volume for production of larger cell masses even if some of these systems have the ability to juxtapose different cell types and cellular stimuli to encourage the growth of more "physiologically" relevant complex cell structures. For the larger volume capable systems (RWVs, scaffold mat and carrier bead bioreactors) there is the limitation of not being able to maintain spatial colocation of appropriate cell types or growth factor stimuli.

Thirdly, the use of constant media flow allows the possibility of conducting long-term culture without the participation of a researcher. It can also allow the analysis of growth factors and metabolites secreted by tumour cells under the influence of various drug administration doses, and delivering additional relevant information to help determine the optimal anti-cancer treatment regimen.

Another goal of the invention is to provide an automated long-term 3D cell culture. Many cell types of importance to medical research require growth periods of several weeks or more. Whilst there are complex automated systems for non-3D cell growth (e.g. engineered stem cells), there are currently no programmable systems that cater for true 3D cell culture at a scale larger than microscope slides. It is also preferable that the system could fit conveniently in a standard laboratory humidified incubator.

Another goal of the invention is to provide an externally automated variable controlled flow and composition of two different cell culture media components. Although examples of pumped single component liquid addition to culture vessels exist already, there is no convenient way to pre-programme for example the pharmacokinetically relevant changing concentrations with time of therapeutic agent (or growth factor) in such a laboratory incubator sized device.

The invention is a cuvette for 3D cell culture comprising a main container comprising means for macroscale 3D growth of cells wherein the cell culture media flow occurs in a substantially vertical direction. The cuvette is closed by two end covers at inlet and outlet end of the main container, connected with cell culture media input and output tubing, wherein fluid transportation ports are placed at the inlet and outlet end covers.

The main container can have the form of a cylinder. The cross-section of the main container can also be a square, hexagon or any other polygon. The main container is preferably made of a transparent material, such as a glass, plastic or quartz, as the growth process within the cuvette can be monitored by an optical detector for example a spectrophotometer. The observed process may show chemiluminescence or fluorescence. The walls of the main container should then be transparent to appropriate wavelengths. Flat walls of the main container facilitate direct observation via microscope, while cylindrical forms of the main container reduce internal reflections of light within the main container. Cylindrical configurations facilitate imaging simply by recording the light or additionally including image processing.

The height of the main container preferably is 5-50 mm and the width of the main container preferably is 3-30 mm. More preferably the height and width of the main container are comprised in the range of: 3-25 mm of width and 5-40 mm of height or 3-20 mm of width and 5-30 mm of height or 4-15 mm of width and 6-25 mm of height or 4-12 mm of width and 8-25 mm of height or 5-10 mm of width and 10-25 mm of height or 6-10 mm of width and 10-20 mm of height, and the most preferably 8-10 mm of width and 12-20 mm of height.

Preferably, the cell culture media flow is quite low, that is 0,005-10 ml/h. Preferably the cell culture media flow can be 0,01-8 ml/h, more preferably 0,05-6 ml/h, more preferably 0,1-4 ml/h, more preferably 0,2-2,5 ml/h, more preferably 0,3-2 ml/h. The most preferable flow rate is 0,5-1 ml/h, as it is intended to effectively support the growth of cells in the hydrogel and thus enable long-term cells growth.

The cuvette may be oriented in any direction, for example horizontally, but it is preferably positioned vertically. The vertical position of the cuvette permits to avoid the problem that i.e. the cells on the bottom would be better supplied by the cell culture media, that occurs when the cuvette is horizontal. A slight deviation in the orientation of the cuvette, even at 45 degrees, does not alter this preferable effect.

The cell culture media flow may preferably occur vertically from top to bottom or bottom to top of the cuvette. Top to bottom flow introduces an additional possibility to study cell metastasis from the tested system by bringing the drain tube to the culture bottle.

The means for macroscale 3D growth of cells can be a hydrogel or a scaffold and they can have fragmented or uniform structure. A uniform structured means for macroscale 3D growth of cells can comprise at least one central vessel conducting the cell culture media through the main container.

Thus the internal structure of the cuvette can be designed so that:
(a) The cells grow in a hydrogel (or other carrier) and the cell culture media flows through the hydrogel to feed the cells with cell culture media.
(b) The cells grow in a hydrogel (or other carrier), and one or more tubes passes through the main container's centre, wherein the cell culture media flows through the tube and the cell culture media spreads mostly by diffusion from the tube to the sides. The tube, called also the central vessel, can be made of collagen, it can also be made of perforated polymers, for example made with electro spinning - it is important that it is permeable and keeps the hydrogel in the right form and stability.
(c) Inside the cuvette there are fragments of the hydrogel (or other carrier) and the cell culture media flows freely in the spaces between randomly arranged fragments of the hydrogel.

A net can be placed at the bottom end of the main container. The net enables uniform distribution of the cell culture media for macroscale 3D cell culture growth. Between the inlet or the outlet of the bottom end cover there should be an empty (without means for 3D cell culture) space allowing for uniform distribution the cell culture media into the main container, that is a container filled entirely with the hydrogel (or other cell culture scaffold) or with hydrogel fragments (or other cell culture scaffolds).

Another aspect of the invention is a cassette for growth and treatment of cells and ease of handling of cell culture outside the sterile cell culture cabinet and re-fitting to the device in the incubator. The cassette comprises at least one container for cell culture growth, a cell culture media reservoir, a reagent reservoir, a waste container, and interconnecting tubes enabling connection with peristaltic pumps. The cassette is detachable and movable as an entity. In the working state, the cassette is placed in a drawer, wherein the reagent reservoir is connected by tubing with a pump connected by tubing with the cuvette, and the cell culture media reservoir is also connected by tubing with another pump connected by tubing with the cuvette. Tubing can be easily detached from the pumps, which preferably are peristaltic pumps. After detaching the tubing from the pumps, the whole cassette can be easily removed from the drawer. The cassette may comprise a detachable holder attached to a side of the cassette suitable to connect the container for cell culture. The shape of the holder will determine what kind of container can be used.

The container for cell culture growth of such cassette can be the cuvette according to the invention or any other suitable container or cell culture system known from the prior art which allows cell culture media to flow through the device and support the growth of cells.

Another aspect of the invention is the whole automated reagent delivery system for growth and treatment of cells, wherein the system comprises
- at least one slide out drawer,
- at least two peristaltic pumps attached to the drawer,
- the cassette according to invention attached to the drawer and allowing connection with two peristaltic pumps, and
- a computer controller and computer program product comprising means for manual and automatic control of liquid flow rates, output composition between the independent liquid reservoirs, pump control and execution of a pre-set liquid flow programs, customisation and saving of new automated liquid handling programs.

Preferably, the two peristaltic pumps are
- a cell culture media pump for pumping cell culture media into the cuvette, and
- a reagent pump for pumping a reagent into the cuvette,
wherein the cell culture media reservoir is connected with the cuvette via a culture media input tubing and a cell culture media pump, and the reagent reservoir is connected with the cuvette via a reagent tubing and a reagent pump. Both pumps preferably provide together constant fluid flow. Pumps can also provide variable flow rate including pulsing flow mimicking heart rate.

The constant fluid flow means that the total amount of fluid passing through the cuvette is constant. Preferably, the cell culture media flow is reduced accordingly when the reagent flow occurs by controlling the operation of the pumps. The tube conducting the fluid with cell culture media and the tube conducting the fluid with reagent are preferably merged into one tube before entering the cuvette to provide one, constant flow of the fluids within the cuvette. As a result of such setup no additional mixers are needed.

After passing through the cuvette, the fluid is directed toward the waste container. It is also possible to use a system of waste containers separating successive fractions.

The invention is presented on figures of the drawing, wherein:
Fig 1 - a view of the system fitted into a conventional laboratory incubator;
Fig. 2 - a view of the system with one of three drawer units pulled out;

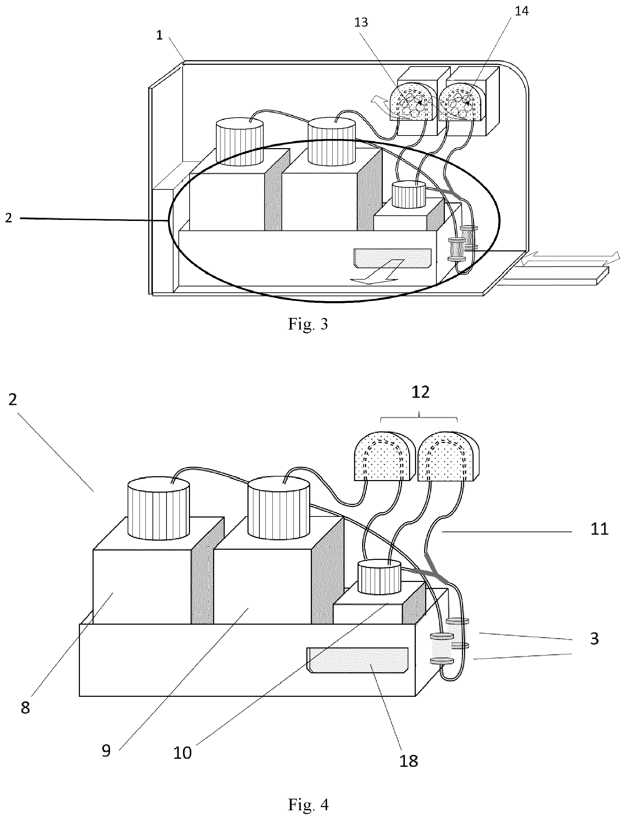

Fig. 3 - a view of the unit comprising a slidable drawer with a removable cassette;
Fig. 4 - a view of the removable cassette;

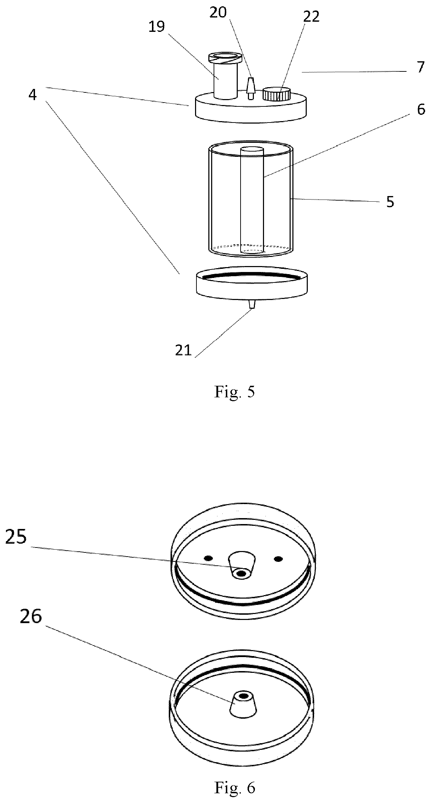

Fig. 5 - an exploded view of the cuvette according to an embodiment of the invention;
Fig. 6 - a view of the cuvette's inlet end cover and outlet end cover;

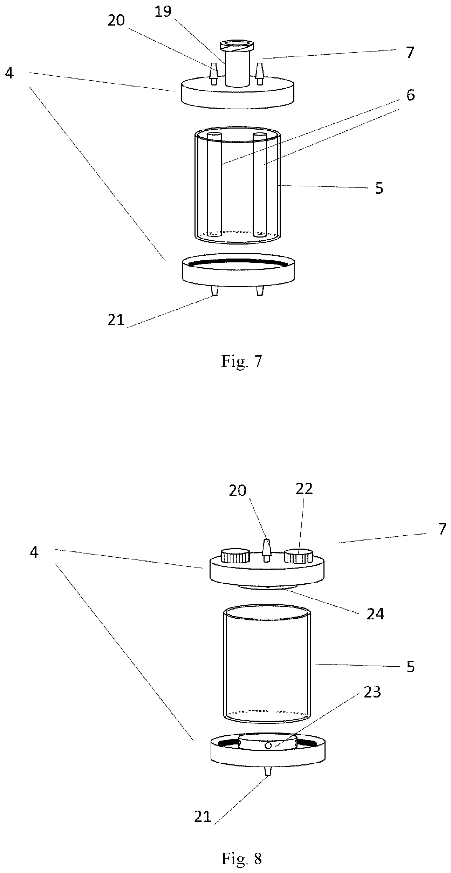

Fig. 7 - an exploded view of the cuvette according to another embodiment of the invention;
Fig. 8 - an exploded view of the cuvette according to another embodiment of the invention.
Fig. 9A - a graph presenting mean plasma concentrations (+ SE) on day 1 after oral administration of imatinib at doses of 400 mg and 500 mg [DOI: 10.1200/JCO.2004.03.050 Journal of Clinical Oncology 22, no. 5 (March 01, 2004) 935-942].
Fig. 9B - a graph presenting a replication of observed plasma concentrations using system according to the invention.

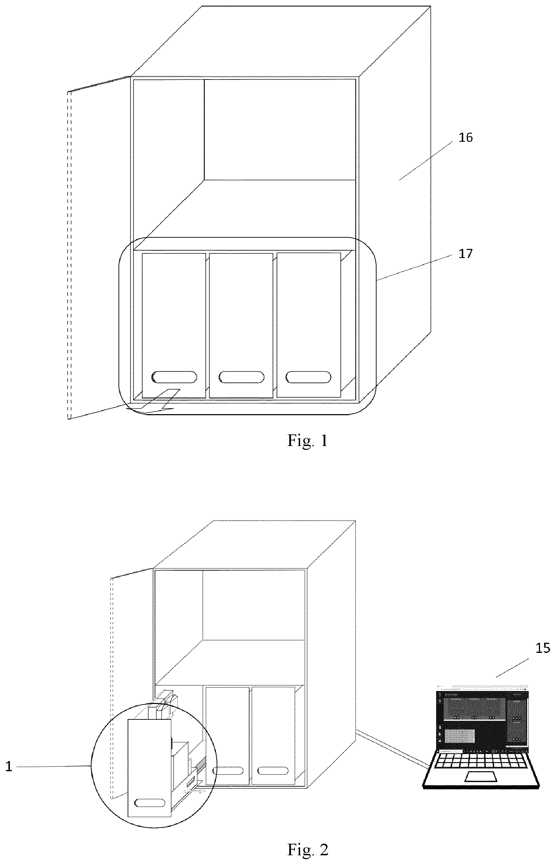

The system is designed to fit in conventional laboratory incubators as shown in Fig. 1. The System comprises at least one and preferably three or six units in a form of slide out drawers, as shown in Fig. 2. Each unit comprises a removable cassette (2) designed so that only one essential component for aseptic handling needs to be transported into an air-flow cabinet for aseptic liquid replacement, additions and disposals, cell or tissue injections and cuvette removal for imaging and further manipulation. Each removable cassette comprises a cell culture media reservoir, a reagent reservoir, at least one cuvette and preferably a waste container, accordingly interconnected by tubing. The cell culture media reservoir is connected with the cuvette via a culture media input tubing and a cell culture media pump for pumping cell culture media into the cuvette, and the reagent reservoir is connected with the cuvette via a reagent tubing and a reagent pump for pumping a reagent into the cuvette. Pumped fluids after passing through the cuvette can be directed via output tubing into the waste container or again into the cell culture media reservoir. Preferably, removable cassette (2) comprises a grab point (18) enabling easy handling. The grab point (18) can be in a form of one or more handles or openings enabling easy grabbing and carrying of the cassette (2), or any other suitable means placed located in any suitable place on the outer surface of the cassette (2).

The cuvette (3) comprises a main container (5) and two end covers at inlet and outlet end of the main container (5), connected with the cell culture media input and output tubing. In the vertical configuration of the container we can call the end overs as a bottom end cover and a top end cover. The two end covers comprise seals that form a liquid and low-pressure resistant connection with the main container (5). Preferably the cuvette (3) comprises means for simple cuvette dis-assembly for retrieval and analysis of contents e.g. via easily removable cuvette's end covers (4).

The top end cover has at least one opening through which cells are injected, forming a top connection point (20) for the cell culture media supply or drain tube. There can be an optional connection (19) for a syringe filter placed at the top end cover - such connection (19) can be used only in the cuvette's versions where the cell culture media flows through the central vessel. Moreover, the same opening through which cells are injected can be used to accommodate a filter that has the purpose of buffering the pressure inside the cuvette - the opening can also be closed with a rubber stopper or a screw, especially when pumping the cell culture media through the hydrogel itself without a tubing, wherein maintaining pressure may be crucial. The central vessel can be connected and stabilized between the bottom and top end cover by mounting elements (25, 26) placed at the inner side of the covers. A bottom connection point (21), for the tube that supplies or drains the nutrient solution, is placed at the bottom end cover. At the top end cover there can be an opening (22) for adding a fill inside the cuvette, be it a hydrogel or injecting cells - this hole can also have a syringe filter mount so that a number of openings is reduced. At the bottom and top end there can be placed a net (23, 24).

The main container (5) is preferably optically transparent (for example is made of glass or plastic) for visual, microscopic, or metabolic imaging.

The central vessel is preferably made of proteins, and preferably transparent or translucent, could be also made from synthetic materials as long as they will allow for easy diffusion of the nutrients through the central vessel wall and supply of oxygen to the surrounding tissue.

Preferably, to provide a means of cell culture media being continuously supplied to and removed from cells growing within the cuvette (3) on or in hydrogel (or other carrier), one or more of the following features are used to form at least one central vessel:
- extracellular matrix component tube(s) (6) ranging from 1 mm to 10 mm diameter to carry cell culture media in and out of cuvette (3), at flow rates that maintain high concentration gradients for diffusion of components to and from growing cells;
- choice of single tube or multiple tubes, with or without opening/shutting valve ability;
- plastic fabric tubing, dialysis tubing.

Such tube(s) (6) support growth of various cell types, facilitating (but not limited to) vascularisation, extravasation, intravasation, and tumour adherence.

It is also possible to provide hydrogel fragments that can simply permeate cell culture media through it instead of through fixed tubes (6). The means for macroscale 3D growth of cells may comprise hydrogel and pre-formed channels in the hydrogel containing inserts.

Flow of cell culture media can be directed via pre-formed channels in hydrogel containing inserts, and stacking of inserts within cuvette (3) facilitates i) increased surface area in contact with pumped oxygen and nutrient containing cell culture media ii) increased volume of hydrogel matrix interspersed with flowing nutrient iii) options for sandwiching of different hydrogel / different juxtaposed cell-type containing layers.

3D cell culture materials known from the prior art include use of animal derived extracellular matrix components (e.g. matrigel, collagen), plant derived 3D scaffolds (e.g. soft agar), synthetic extracellular matrix-like components (e.g. synthetic laminin, collagen, fibronectin), synthetic soft 3D cellular scaffolds (e.g. Peptide modified polyacrylamide gels), or synthetic hard 3D scaffolds (e.g. alvetex). The majority of the above materials can be well defined chemically and functionally, the exception being the variation seen from batch to batch of animal derived basement membrane preparations (variations in endogenous growth factor levels and possibly uncharacterised virus or antigen presence that may affect long term comparative experimental studies and in particular jeopardise animal studies). In general, 3D cell cultures according to the prior art are either grown by seeding on the surface of a matrix, by seeding within the body of a matrix, or by seeding in suspension cultures (whose volumes are often physically constrained).

Current preparation of 3D cell culture material for use in the desired experimental device may include: warming of extracellular matrix components to catalyse gel support formation, cooling of soft agars to permit gel formation, and chemical or photo cross-linking of scaffold components in situ or prior to dispensing scaffold. A protein hydrogel as described in documents EP3689971A1 and WO2020161613A1 can be used. The above procedures often allow pre-mixing of liquid components with cells prior to gel formation; the nature of the chemical or photo cross-linking determines whether or not cells can be added before gel formation. In the case of animal, plant and synthetic soft scaffold materials, each can normally be dispensed into the appropriate cell culture receptacle using conventional liquid handling techniques. In the case of synthetic hard cellular scaffolds these may be produced using techniques such as 3D-printing into culture receptacles or adding pieces of pre-formed scaffold as inserts into the culture receptacle.

The tubes with the cell culture media (supplying and discharging the cell culture media) can be attached to the cuvette (3) with the use of sleeves, wherein the inner diameter of the sleeve is slightly larger than the inner diameter of the tube supplying the nutrient solution.

Such cuvette (3) can be used for the cultivation of adherent cells or cells immobilized in a hydrogel or hydrogel fragments, wherein the cells can be human, animal, insect, or plant cells, or their combinations - additionally, bacterial cells can be added, especially when cell culture creates a research model, e.g. bacterial infection.

The cuvette (3) can be attached to the cassette (2) via main and bottom grips of the cassette (2) that fit closely to the tube mounting sleeves - such mounts are centrally located in relation to the axis of rotation of the cuvette, thanks to which its imaging will be more precise.

There is also a possibility to use any other suitable container or cell culture system known from the prior art which allows for cell culture media to flow through the device and support the growth of cells, instead of the cuvette (2) according to the invention.

The cassette (2) preferably has a positioning system, for example guides and magnets on the sides that position it within the drawer. Precisely repeatable positioning of the cassette (2) within the drawer is important for the imaging system performance.

The cell culture media reservoir, the reagent reservoir, and the waste container preferably comprise filters (e.g. 0.2 µm) preferably placed in their covers. Filters provide sterility inside the bottle, but also give the possibility of stabilizing the pressure in the bottle when the cell culture media is pumped into it and pumped out of the reservoirs and containers.

The cassette's (2) design facilitates sterile work. It allows for preparation of the test system (opening and closing of cuvette, reservoirs) under sterile conditions (in the air flow cabinet), and transporting it (in non-sterile conditions) to the device containing pumps. All elements are compactly arranged in one place, which facilitates the ergonomic handling of the entire cassette (2). It is also possible to connect the cassette (2) to the pumps in a non-sterile incubator while maintaining the sterility of the entire internal culture system.

The automated reagent delivery system for growth and treatment of cells according to the invention comprises at least one slide out drawer (1), at least two peristaltic pumps attached to the drawer, and the cassette (2) attached to the drawer (1) and connected with the peristaltic pumps, a computer controller and computer program product (15). The computer program product comprises means for manual and automatic control of liquid flow rates, output composition between the independent liquid reservoirs, pump control and execution of pre-set liquid flow programs, customization and saving of new automated liquid handling programs. There is a possibility of digitally saving complete programmed segments of the pumping protocol, e.g. in 24-hour segments. There is also a possibility of recording all flow data - the electronics are coupled in such a way that it allows one to plan everything from the very beginning to the very end. It is a huge advantage over other systems that, for example, require human intervention in order to complete a long-term experiment with the risk of errors.

While the system is in use, first of all, a program is created and the experiment plan is electronically recorded. It can also simply be copied and used or, in addition, it can be edited and modified for future experiments. The software is created as "blocks" - elements of the experiment that can be freely copied / modified / saved. These features provide a rapid way to create, modify and execute experiments without error.

In the event of a power failure the system will restart in the same place after power resumes, and the information about the power outage will be included in the final experiment report automatically generated by the program.

The pumps used in the system can be a cell culture media pump for pumping cell culture media into the cuvette, and a reagent pump for pumping a reagent into the cuvette. The cell culture media reservoir is thus connected with the cuvette via a culture media input tubing and a cell culture media pump, and the reagent reservoir is connected with the cuvette via a reagent tubing and a reagent pump. Preferably, both pumps provide together a constant fluid flow. The mixing of the reagent fluid with cell culture media occurs in the overlapping segment of the input tubes before entering the cuvette.

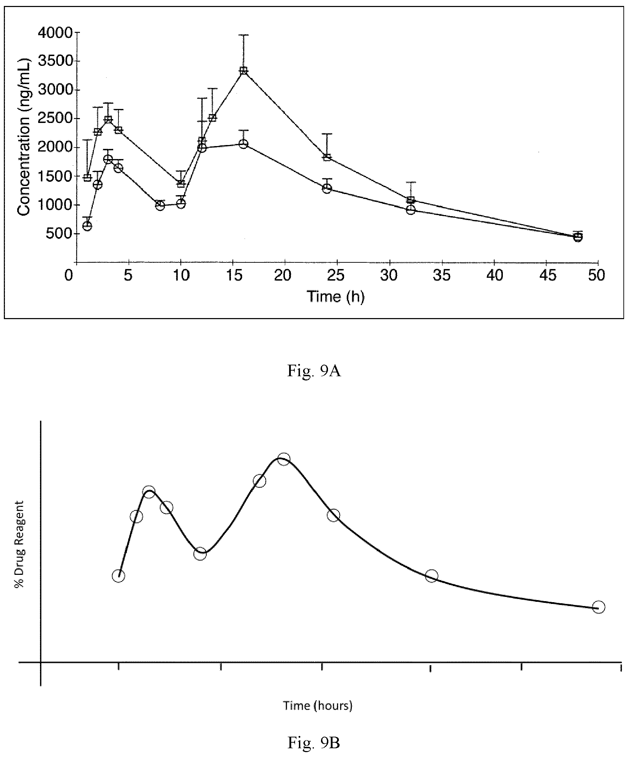

The performance of the system in visible on graphs on Fig. 9A and 9B. The graph of Fig. 9A presents mean plasma concentrations (+ SE) on day 1 after oral administration of imatinib at doses of 400 mg and 500 mg bid. The x-axis shows the time in hours (h). The curve marked with circles (○) presents results for 400 mg dose and the curve marked with squares (□) presents results for 500 mg dose. The source of the graph is DOI: 10.1200/JCO.2004.03.050 Journal of Clinical Oncology 22, no. 5 (March 01, 2004) 935-942. The observed plasma concentrations were replicated using the system according to the invention. Results of mimicked pharmacokinetics are presented in Fig. 9B.

## Claims

1. Cuvette (3) for 3D cell culture growth comprising
a main container (5) comprising means for macroscale 3D growth of cells, **characterised in that** it further comprises
two end covers (4) at inlet and outlet ends of the main container (5), connected with cell culture media input and output tubing, wherein fluid transportation ports (7) placed at the inlet and outlet end covers (4).

2. Cuvette (3) according to claim 1, wherein the main container (5) is a cylinder or has a polygon-shaped cross-section.

3. Cuvette (3) according to any of the preceding claim, wherein the height of the main container (5) is 5-50 mm and the weight of the main container (5) is 3-30 mm, preferably wherein the height and width of the main container are comprised in the range of: 3-25 mm of width and 5-40 mm of height or 3-20 mm of width and 5-30 mm of height or 4-15 mm of width and 6-25 mm of height or 4-12 mm of width and 8-25 mm of height or 5-10 mm of width and 10-25 mm of height or 6-10 mm of width and 10-20 mm of height, and the most preferably 8-10 mm of width and 12-20 mm of height.

4. Cuvette (3) according to any of the preceding claim, wherein the cell culture media flow is 0,005-10 ml/h, preferably 0,01-8 ml/h, more preferably 0,05-6 ml/h, more preferably 0,1-4 ml/h, more preferably 0,2-2,5 ml/h, more preferably 0,3-2 ml/h, and the most preferably 0,5-1 ml/h.

5. Cuvette (3) according to any of the preceding claim, wherein the cell culture media flow occurs in a direction tilted between +45 to -45 degrees from the vertical direction, and preferably the cell culture media flow occurs vertically and preferably the cell culture media flow occurs from top to bottom or bottom to top of the cuvette (3).

6. Cuvette (3) according to any preceding claim, wherein the means for macroscale 3D growth of cells is hydrogel or scaffold.

7. Cuvette (3) according to claim 6, wherein the means for macroscale 3D growth of cells comprises at least one central vessel for conducting the cell culture media through the main container (5).

8. Cuvette (3) according to claim 6, wherein the means for macroscale 3D growth of cells is in a form of fragments or has uniform structure.

9. Cuvette (3) according to claim 8, comprising a net at bottom end.

10. Cassette (2) for growth and treatment of cells comprising
at least one container for cell culture growth,
a cell culture media reservoir,
a reagent reservoir,
a waste container, and
interconnecting tubes enabling connection with peristaltic pumps,
**characterised in that** the cassette (2) is detachable and movable as an entity.

11. Cassette (2) according to claim 10, wherein the container for cell culture growth is the cuvette (3) according to any of claims 1-11.

12. Cassette (2) according to claim 10 or 11, further comprising a positioning system.

13. Automated reagent delivery system for growth and treatment of cells, wherein the system comprises
at least one slide out drawer (1),
at least two peristaltic pumps attached to the drawer,
the cassette (2) according to any of claims 10 -12 attached to the drawer (1) and connected with the peristaltic pumps,
a computer controller and computer program product (15) comprising means for manual and automatic control of liquid flow rates, output composition between the independent liquid reservoirs, pump control and execution of pre-set liquid flow programs, customisation and saving of new automated liquid handling programs.

14. System according to claim 13, wherein the two peristaltic pumps are
a cell culture media pump for pumping cell culture media into the cuvette, and
a reagent pump for pumping a reagent into the cuvette,
wherein the cell culture media reservoir is connected with the cuvette via a culture media input tubing and a cell culture media pump,
and the reagent reservoir is connected with the cuvette via a reagent tubing and a reagent pump,
and both pumps provide together constant or pulsating fluid flow.

15. Use of the cuvette according to any of claims 1-9 or the cassette according to any of claims 10-12 or the system according to any of claims 13-14 for mimicking pharmacokinetics or in vivo ADME processing of a drug.
